# EUROPEAN PATENT APPLICATION

(11) **EP 4 686 877 A1**
(43) Date of publication of application: **04.02.2026**
(21) Application number: 24192345.7
(22) Date of filing: 01.08.2024
(51) Int. Cl.: F24C 15/20, B01D 53/00, A61L 9/20

(54) **FILTER FOR A VENTILATION SYSTEM**

(71) Applicant: Randolph Beleggingen B.V., 3446 GK Woerden (NL)
(72) Inventor: VIANEN, Lisa Melanie, 3446 CK Woerden (NL)
(74) Representative: Nederlandsch Octrooibureau

(57) **Abstract**

This disclosure relates to a filter for a kitchen ventilation system. The filter comprises UV-C lighting, wherein the UV-C lighting is configured to produce UV-C light, wherein an intensity of the UV-C light is adjustable; an airflow sensor, configured to measure data related to an airflow generated by the kitchen ventilation system; and a controller, configured to: receive a signal from the airflow sensor, wherein the signal relates to measurement data regarding an airflow generated by the kitchen ventilation system; based on the signal, determine whether the kitchen ventilation system is operating, and if the controller determines that kitchen ventilation system is operating: determine an airflow generated by the operating kitchen ventilation system; and control the UV-C lighting to produce UV-C light, wherein the intensity of the UV-C light is based on the airflow generated by the kitchen ventilation system (200).

## Description

### Field of the invention

This disclosure relates to a filter for a (kitchen) ventilation system, in particular to a grease filter comprising an ultraviolet (UV) lamp.

### Background art

Kitchen ventilation systems are used to remove cooking fumes from kitchens. Particularly in professional kitchens, which may be used for the entire day, it is important that the ventilation system functions well. Cooking fumes may comprise grease and/or odours. To prevent clogging of the kitchen ventilation system and/or to reduce the amount of exhausted odours, it may be beneficial to filter the air in the kitchen ventilation system.

The above description is not necessarily prior art publicly known before the present application was filed.

### Summary of the invention

### Technical problem

To filter the air, a UV filter may be used. Such a UV filter exposes the air inside the ventilation system to UV-C light. The UV-C light may cause various chemical reactions to take place. For example, the UV-C light may cause oxygen in the air to react into ozone. This ozone may then react with odorous molecules, causing these to break down, after which these can be drained away. For another example, the UV-C light may cause grease to decompose directly.

In order to filter the exhaust air to a satisfactory degree, there should be a sufficient amount of ozone and the ozone should be given some time to react. For example, the ozone may require two seconds to react with the exhaust air. Similarly, the grease should be exposed to a sufficient amount of UV-C light. The total size of the filter and the amount of light the filter should produce is determined by the size and airflow of the kitchen ventilation system.

However, the system may be expensive to operate and/or require a significant amount of maintenance.

### Technical solution

This application addresses the problem of optimizing the use of UV-C lighting in a kitchen ventilation system. In particular, this application relates to reducing operational costs and/or maintenance costs of a UV-C lighting filter.

The application provides solutions for adjusting the intensity of the UV-C light based on the airflow generated by the kitchen ventilation system, ensuring efficient and effective air filtration while minimizing energy consumption.

Embodiments of the invention are associated with various advantages and/or technical effects.

In a first aspect of the invention, there is disclosed a filter for a kitchen ventilation system, comprising: UV-C lighting, wherein the UV-C lighting is configured to produce UV-C light, wherein an intensity of the UV-C light is adjustable; an airflow sensor, configured to measure data related to an airflow generated by the kitchen ventilation system; a controller, configured to: receive a signal from the airflow sensor, wherein the signal relates to measurement data regarding an airflow generated by the kitchen ventilation system; based on the signal, determine whether the kitchen ventilation system is operating, and if the controller determines that kitchen ventilation system is operating: determine an airflow generated by the kitchen ventilation system; control the UV-C lighting to produce UV-C light, wherein the intensity of the UV-C light is based on the airflow generated by the kitchen ventilation system.

The adjustable intensity of the UV-C lighting allows for energy-efficient operation by using only the necessary amount of UV-C light to produce ozone and/or to remove grease based on the ventilation system's activity, thereby reducing power consumption.

The integration of an airflow sensor with the controller enables real-time monitoring and response to changes in the kitchen ventilation system's airflow, ensuring optimal performance of the filter system.

By dynamically adjusting the UV-C light intensity in response to the ventilation system's operation, the filter can maintain effective air purification while minimizing the wear on the UV-C lighting components, extending their lifespan.

The filter provides the advantage of controllability, as the intensity of the UV-C lighting can be adjusted based on the airflow generated by the kitchen ventilation system.

The filter offers the advantage of performance, as the UV-C lighting produces ozone to effectively filter and purify the air in the kitchen ventilation system.

The filter allows for monitoring, as it includes an airflow sensor that measures data related to the airflow generated by the kitchen ventilation system, providing real-time information to the controller. For example, the airflow sensor may measure a pressure, from which an airflow can be computed.

The filter ensures safe and efficient operation, as the controller determines whether the kitchen ventilation system is operating and controls the UV-C lighting accordingly.

The filter reduces the amount of ozone in the exhaust air, because the production of ozone is scaled down when less ozone is needed.

Advantageously, the controller is further configured to control the UV-C lighting to produce light at a first intensity if the airflow generated by the kitchen ventilation system is below a first predetermined airflow.

The ability to produce light at a first intensity for lower capacities enhances the filter's adaptability to varying kitchen conditions, ensuring efficient grease removal and/or ozone production without overuse of the UV-C lighting at times of low ventilation activity.

By conserving energy at lower capacities, the filter contributes to the overall sustainability of the kitchen environment, potentially reducing the carbon footprint associated with the operation of the ventilation system.

Advantageously, the controller is further configured to control the adjustable UV-C lighting to produce light at at least a second intensity if the airflow generated by the kitchen ventilation system is above a second predetermined airflow, wherein the second predetermined airflow is greater than or equal to the first predetermined airflow.

The provision for at least a second intensity level allows for a tailored air purification process that can be intensified for higher ventilation capacities, ensuring that air quality is maintained even under heavy kitchen use.

The ability to adjust the UV-C lighting to a higher intensity when needed provides flexibility in managing varying levels of cooking fumes, odours, and airborne contaminants, enhancing the overall effectiveness of the kitchen ventilation system.

The controller's capability to differentiate between different operational airflows and adjust the UV-C lighting accordingly helps to prevent the underutilization or overexertion of the filter system, optimizing its performance and reliability.

Advantageously, the second intensity is less than a maximum intensity of the UV-C lighting, wherein the second predetermined airflow is equal to the first predetermined airflow, wherein the controller is further configured to control the adjustable UV-C lighting to produce light at the maximum intensity, when the kitchen ventilation system is operating above a third predetermined airflow, wherein the third predetermined airflow is greater than the second predetermined airflow.

The configuration to utilize a second intensity that is less than the maximum allows for a graded approach to air purification, which can be more energy-efficient than operating at maximum intensity at all times.

The ability to reserve the maximum intensity for when the kitchen ventilation system is operating above a third predetermined airflow ensures that the highest level of air purification is available when most needed, such as during peak cooking times.

By setting the second predetermined airflow equal to the first, the filter system simplifies the operational thresholds, making it easier to design control algorithms and potentially reducing the complexity of the system's electronics.

Advantageously, an intensity of the UV-C light is proportional to the airflow generated by the kitchen ventilation system.

The proportional relationship between UV-C light intensity and ventilation airflow ensures a consistent level of grease removal and/or ozone production relative to the amount of air being processed, which can lead to more uniform exhaust air quality.

By automatically adjusting the intensity of the UV-C light, the filter system can respond quickly to sudden changes in ventilation demand, such as when cooking activity increases or decreases, maintaining optimal air purification at all times.

Advantageously, the UV-C lighting comprises a plurality of UV-C lamps, wherein, when the UV-C lighting produces light at the first intensity, a first fraction of the plurality of UV-C lamps is switched on and a first remainder of the plurality of UV-C lamps is switched off, and wherein, when the UV-C lighting produces light at the second intensity, a second fraction of the plurality of UV-C lamps is switched on and a second remainder of the plurality of UV-C lamps is switched off.

Implementing the UV-C lighting in the form of a plurality of UV-C lamps is particularly convenient. For typical professional kitchen ventilation systems, a required power of UV-C lighting may be around a kilowatt, or more. To provide adjustable UV-C lighting of this airflow, using a plurality of non-dimmable lamps, for example lamps with a fixed power between 50 W and 100 W, may be an economical option. Non-dimmable lamps may be cheaper than dimmable lamps. Furthermore, selectively switching off some non-dimmable lamps may be more efficient and/or contribute to longer life times, compared to dimming dimmable lamps. Because, in practice, professional kitchen ventilation systems may require at least 10 UV-C lamps in order to have a sufficient airflow, selectively switching these on and off may provide a sufficient degree of granularity in terms of possible amounts of UV-C light.

The ability to adjust the number of UV-C lamps that are switched on allows for variable intensity levels, which can be tailored to the specific purification needs at any given time, thereby enhancing the efficiency of the UV-C lighting system.

By selectively switching on a fraction of the UV-C lamps, energy consumption can be optimized, leading to cost savings and a reduced environmental impact over the lifetime of the filter system.

The selective operation of UV-C lamps can reduce the thermal load on the filter and surrounding components, potentially extending the overall lifespan of the kitchen ventilation system.

The selective operation of UV-C lamps may lead to a reduction in burning time for individual UV-C lamps. This can extend the overall lifespan of these individual lamps.

Advantageously, the controller is further configured to determine which of the UV-C lamps to switch on or off, based on an expected lifetime of the UV-C lamps.

The controller's ability to determine which UV-C lamps to switch on or off based on their expected lifetime can ensure even usage among the lamps, thereby enhancing the lifespan of the entire UV-C lighting array.

By managing the operational life of each UV-C lamp, the controller can help maintain consistent purification performance over time, reducing the risk of decreased efficacy due to aging lamps.

The proactive management of lamp usage can lead to fewer maintenance requirements and lower operational costs, as lamps can be replaced in a more coordinated and efficient manner.

Furthermore, reducing the maintenance requirements may enhance safety. Because the filter includes UV-C lighting and produces ozone, there may be some inherent danger in maintaining the filter. Even though safety precautions may be taken to reduce the risk of the filter being switched on during maintenance, reducing the number of times the filter needs to be opened may nevertheless be beneficial.

Advantageously, the controller is further configured to, if the controller determines that the kitchen ventilation system is not operating, switch off the UV-C lighting.

Automatically switching off the UV-C lighting when the kitchen ventilation system is not operating prevents unnecessary energy usage, contributing to energy conservation and cost savings.

Automatically switching off the UV-C lighting when the kitchen ventilation system is not operating enhances the lifespan of the UV-C lighting.

The controller's ability to turn off the UV-C lighting in the absence of ventilation can prevent the accumulation of ozone or other byproducts that might otherwise occur if UV-C light were to operate in a stagnant air environment.

This feature enhances safety by ensuring that the UV-C lighting is only active when it is needed, reducing the potential for accidental exposure to UV-C radiation when the kitchen ventilation system is not in use.

Advantageously, the controller is operably connected to the kitchen ventilation system, and wherein the controller is further configured to determine whether the kitchen ventilation system is operating based on a signal received from the kitchen ventilation system.

The controller's direct connection to the kitchen ventilation system allows for real-time monitoring and response to the operational status of the ventilation, ensuring that the UV-C lighting is synchronized with the system's needs.

By relying on a signal to determine the status of the kitchen ventilation system, the controller can quickly and accurately control the UV-C lighting, improving the overall responsiveness and efficiency of the system.

The additional direct connection may enhance safety. Because the controller can check in two different ways whether the system is operating, or not, the controller can ensure that the lighting is switched off if either the signal or the airflow sensor indicates that the kitchen ventilation system is not operating. In this way, it may be prevented that the UV-C lighting is on during cleaning and/or maintenance of the filter.

A second aspect of the invention relates to a kitchen ventilation system, comprising a filter according to the first aspect of the invention.

A kitchen ventilation system that includes a filter with the aforementioned features can provide a comprehensive solution for air purification.

The inclusion of a filter with variable UV-C lamp intensity and smart lamp management allows the kitchen ventilation system to adapt to varying levels of kitchen activity and contamination, ensuring optimal performance at all times.

The system's ability to automatically regulate the UV-C lighting based on the operation of the ventilation system contributes to a safer kitchen environment by minimizing unnecessary exposure to UV-C light and reducing the production of harmful byproducts.

Third, fourth and fifth aspects of the invention relate to a method of filtering kitchen exhaust air to a related computer program product, and to a memory storing such a computer program product. The method may be carried out using a filter according to the first aspect. The method involves receiving a signal from an airflow sensor, wherein the signal relates to measurement data regarding an airflow generated by the kitchen ventilation system; based on the signal, determining whether a kitchen ventilation system is operating, and if it is determined that kitchen ventilation system is operating: determining an airflow generated by the operating kitchen ventilation system; and controlling UV-C lighting to produce UV-C light, wherein the intensity of the UV-C light is based on the airflow generated by the kitchen ventilation system.

Such a method can allow for the efficient operation of a filter for a kitchen ventilation system.

### Brief description of drawings

Embodiments of the present invention will be described hereinafter, by way of example only, with reference to the accompanying drawings which are schematic in nature and therefore not necessarily drawn to scale. Furthermore, like reference signs in the drawings relate to like elements and a repeated description related thereto may be omitted. In the attached figures,
- Figure 1 schematically shows a filter for a kitchen ventilation system,
- Figure 2 schematically shows a kitchen ventilation system with a filter.

### Detailed description

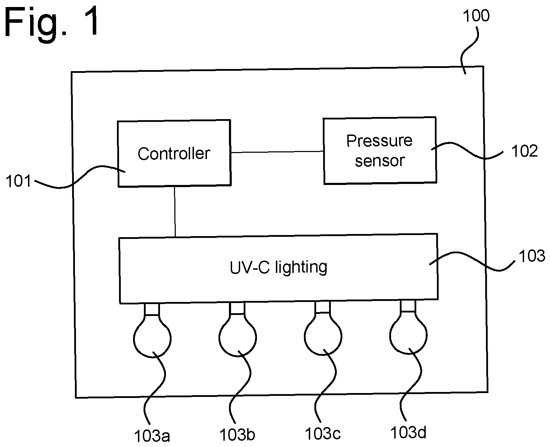

Figure 1 schematically shows a filter 100. The filter is for a kitchen ventilation system.

The filter 100 comprises a controller 101, a pressure sensor 102, and UV-C lighting 103. The UV-C lighting 103 comprises a plurality of UV-C lamps 103a, 103b, 103c, and 103d.

The controller 101 is operably connected to the pressure sensor 102 and the UV-C lighting 103. The controller 101 is configured to control the overall operation of the filter 100, and is in particular configured to control the UV-C lighting 101 based on a signal from the pressure sensor 102.

The pressure sensor 102 is operably connected to the controller 101. The pressure sensor 102 is configured to detect a pressure generated by a kitchen ventilation system. In operation, the kitchen ventilation system is suitable, intended and configured for exhausting cooking fumes, and may be suitable, intended and/or configured for bringing in fresh air. So, there will be an airflow of cooking fumes inside the kitchen ventilation system, in particular in an exhaust duct of the kitchen system. In order to cause this airflow, the kitchen ventilation system may comprise a motor configured to reduce the pressure in the exhaust duct. If the pressure inside the exhaust duct is lower than the pressure proximal to a cooking place, cooking fumes will be sucked into the exhaust duct and cause an airflow to the exhaust duct. The pressure in the exhaust duct may be linearly related to the volume of the airflow. So, by measuring the pressure in the exhaust duct, the volume of the airflow may be determined. The pressure sensor 102 may comprise computing circuitry for converting the pressure to the volume of the airflow and/or vice versa. The pressure sensor 102 may be any kind of device and/or sensor suitable for detecting a pressure and/or (a volume of) an airflow, such as a pressure and/or an airflow generated by a kitchen ventilation system.

The pressure sensor 102 is configured to send a signal indicating a detected pressure to the controller 101. This signal may, for example, directly indicate a measurement value for the pressure, or a related value, such as, for example, an airflow corresponding to a measured pressure. The signal may be transmitted in one or more of various ways known to the skilled person. Based on the signal, the controller 101 obtains the airflow and may compare the airflow to a maximum airflow of the kitchen ventilation system. This maximum airflow may be preconfigured and stored in a memory of the controller. For example, the maximum airflow of the kitchen ventilation system can be preconfigured into the memory of the controller 101 upon installation of the filter 100 into the kitchen ventilation system. The controller 101 may determine the airflow as a proportion and/or percentage of the maximum airflow.

The airflow of the kitchen ventilation system may be referred to as a airflow generated by the kitchen ventilation system. The maximum airflow of the kitchen ventilation system may be referred to as a maximum airflow generated by the kitchen ventilation system.

The UV-C lighting 103 is operably coupled to the controller 101. The UV-C lighting 103 is adjustable. The UV-C lighting 103 may comprise a plurality of UV-C lamps 103a, 103b, 103c, 103d.

The UV-C lighting 103 is configured to produce UV-C light. The UV-C light may cause grease in the exhaust air to react, thereby removing the grease. Furthermore, the UV-C light may produce ozone. The ozone can then also react with various substances in the exhaust air, and may, for example, reduce odours.

The UV-C lighting may be made adjustable through comprising one or more dimmable UV-C lamps 103a-103d, or through comprising a plurality of UV-C lamps 103a-103d, which may be dimmable or non-dimmable, wherein control of at least some of the plurality of the UV-C lamps 103a-103d is independent from at least some other of the plurality of the UV-C lamps 103a-103d. For example, the UV-C lighting 103 may comprise individually controllable non-dimmable lamps 103a-103d.

The controller 101 is configured to control the UV-C lighting 103.

The controller 101 determines whether the kitchen ventilation system is operating. The controller 101 may determine whether the kitchen ventilation system is operating in various ways.

If the controller 101 detects, for example using the pressure sensor 102, the presence of an airflow, the controller 101 may conclude that the kitchen ventilation system is operating. To account for measurement errors, a minimum airflow may be configured and the controller 101 may determine that the kitchen ventilation system is operating only if the airflow exceeds the minimum airflow, while, if the airflow does not exceed the minimum airflow, the controller 101 may determine that the kitchen ventilation system is not operating.

Additionally, the controller 101 may be operably connected to a motor of the kitchen ventilation system. In particular, the controller 101 may be electrically connected to the motor, more in particular, such that the controller 101 detects whether an electrical current is flowing through the motor. In this case, the controller 101 is informed directly whether the motor is operating or not. If the motor is operating, the controller 101 may determine that the kitchen ventilation system is operating. If the motor is not operating, the controller 101 may determine that the kitchen ventilation system is not operating.

Additionally, the controller 101 may receive a user input indicating whether the kitchen ventilation system is operating or not, and determine accordingly.

The skilled person appreciates that both UV-C light and ozone are harmful to humans. Therefore, having one, or preferably multiple, check in the system for determining whether the kitchen ventilation system is operating, or not, may contribute to enhancing safety, because the controller may be configured to only switch the UV-C lighting on if it is determined with sufficient certainty that the kitchen ventilation system is operating.

Furthermore, the filter 100 may include further safety features to prevent exposure to UV-C light and/or ozone during cleaning and/or maintenance. For example, the filter 100 may be provided with a housing operably connected to the UV-C lighting 103, for example via a switch, such that, when the housing is opened for maintenance, an electricity supply to the UV-C lighting 103 is blocked.

If the controller 101 determines that the kitchen ventilation system is not operating, the controller 101 may switch the UV-C lighting 103 off. This may contribute to safety, may save energy, and/or may extend the lifespan of the UV-C lighting 103.

If the controller 101 determines that the kitchen ventilation system is operating, the controller 101 determines the airflow and controls the UV-C lighting 103 to produce UV-C light, wherein the intensity of the UV-C light is based on the airflow generated by the kitchen ventilation system.

It should be appreciated that the determination of whether the kitchen ventilation system and of the airflow can be done in a single step. For example, the controller 101 may determine the airflow based on measurement data once and use this value both for determining whether the kitchen ventilation system is operating (for example, by checking that the airflow exceeds zero or a minimum airflow value), and for controlling the intensity of the UV-C light. This could, for example, be implemented by always having the controller 101 control the intensity of UV-C light based on the airflow and by configuring the controller 101 to control the intensity of UV-C light to be zero (that is, to switch the UV-C lighting 103 off) in case that the airflow is zero or below a minimum airflow value.

Depending on the configuration of the UV-C lighting 103, the controller 101 may be configured to control the intensity of the UV-C light proportionally and/or in a discrete manner, based on the airflow.

For example, if the UV-C lighting 103 comprises one or more dimmable lamps 103a-103d, the controller 101 may be configured to control the UV-C light to be proportional to the airflow.

For another example, if the UV-C lighting 103 comprises a plurality of lamps 103a-103d, some of which controllable independently from at least some others, the controller 101 may be configured to selectively switch some UV-C lamps 103a-103b on or off.

The UV-C lighting 103 has a maximum intensity. This maximum intensity may be chosen to ensure a sufficient grease filtering and/or production of ozone at a maximum airflow that the kitchen ventilation system can generate. That is, the maximum intensity of the UV-C lighting is adapted to the specific kitchen ventilation system in which the filter 100 is intended to be used.

In addition, the precise arrangement of the UV-C lighting 103, which may for example comprise a number of lamps 103a-103d arranged at certain positions in the filter, as well as an overall shape of the filter 100, may be adapted to the specific kitchen ventilation system in which the filter 100 is intended to be used.

The skilled person knows how to choose the maximum intensity, the number and arrangement of lamps, and the overall shape of the filter 100, based on the specific kitchen ventilation system.

In practice, this means that the UV-C lighting 103 may comprise at least around 5 UV-C lamps. For example, for a kitchen ventilation system with a maximum airflow of around 10⁴ m³/h, a maximum intensity of around 1.6 kW may be appropriate. Such a maximum intensity of 1.6 kW may be provided for by using 20 UV-C lamps of 80 W.

Because the number of UV-C lamps that is typically used may be relatively large, selectively switching small numbers of these lamps on or off may still allow for relatively fine grained control. So, even if the lamps are not dimmable, fine-grained may be possible. On the other hand, using non-dimmable lamps may have certain advantages. For example, non-dimmable lamps may be easier, and hence cheaper, to produce. Furthermore, if a lamp is completely switched off, electricity use and wear may be negligible, while, if a lamp is merely dimmed, electricity use and/or wear may be lower than when the lamp is used at full power, but not necessarily proportionally so. In other words, selectively switching off some lamps may achieve the same intensity as dimming lamps, while achieving a lower total power consumption and/or a higher lifetime. In particular, when selectively switching off lamps, the controller may be configured to vary which lamps are switched on or off first, in order to distribute the total burning hours and/or the total number of times lamps are switched on or off relatively equally among the lamps. By dividing the load among the lamps in this way, the total life time of the UV-C lighting 103 may be extended.

The controller 101 may be configured to control the intensity of the UV-C lighting 103, for example by selectively switching on and off UV-C lamps, in a variety of ways. By optimizing this control, energy may saved, maintenance may be lowered, and/or a total effective lifetime may be extended, all while still achieving a sufficient filtering performance.

To give a concrete example, suppose that there are 12 UV-C lamps. The controller 101 may be configured to switch the lamps on or off in groups of 4. For example, if the controller 101 determines that the kitchen ventilation system is operating and an airflow is 0-34% of the maximum airflow, 4 lamps may be switched on and 8 may be switched off. If the airflow is 35-66%, 8 lamps may be switched on and 4 may be switched off. If the airflow is 67-100%, all 12 lamps may be switched on. So, if the kitchen ventilation system is operating below a first threshold of 34%, only 4 lamps are switched on. If the kitchen ventilation system is operating above the first threshold but below a second threshold of 67%, 8 lamps are switched on. If the kitchen ventilation is operating above the second threshold, all lamps are switched on.

To give another concrete example, suppose that there are 10 UV-C lamps. The controller 101 may be configured to control the number of lamps to be switched on depending on the airflow relative to the maximum airflow approximately proportionally, according to the following table:

| **Airflow (% of max. airflow)** | **# Lamps switched on** |
|---|---|
| 0-4 | 0 |
| 5-14 | 1 |
| 15-24 | 2 |
| 25-34 | 3 |
| 35-44 | 4 |
| 45-54 | 5 |
| 55-64 | 6 |
| 65-74 | 7 |
| 75-84 | 8 |
| 85-94 | 9 |
| 95-100 | 10 |

This example illustrates that a minimum threshold may be used before the UV-C lighting 103 is switched on. So, if the airflow is 4% of the maximum airflow or less, the controller 101 may determine that the kitchen ventilation system is not operating. This allows to account for measurement errors. Furthermore, this example illustrates that, if the number of UV-C lamps is sufficiently large and especially if the UV-C lamps are individually controllable, the light intensity may be controlled in a fine grained manner, for example to make the light intensity proportional to the airflow.

To give yet another concrete example, suppose that there are 5 lamps. If the controller 101 determines that the kitchen ventilation system is operating and an airflow is 0-30% of the maximum airflow, 2 lamps may be switched on and 3 may be switched off. If the airflow is 31-45%, 3 lamps may be switched on and 2 may be switched off. If the airflow is 46-100%, all 5 lamps may be switched on. So, the thresholds need not be exactly proportional to the number of lamps and the number of additional lamps that is switched on at each threshold need not be equal at each threshold. For example, to ensure sufficient performance at low airflows, it may be desirable if the light intensity is slightly higher than it would be under proportional control. A disadvantage of this approach is that the energy and maintenance savings may, however, be more limited.

The skilled person appreciates that the above examples are merely for showing certain possibilities for control, but not limiting in any way. The controller 101 may be configured to control the UV-C lighting 103 based on the airflow in any appropriate way.

Although Figure 1 depicts the controller 101, the pressure sensor 102, and the UV-C lighting 103 as distinct components, it should be appreciated that various components may be integrated into a single component. For example, the controller 101 may be integrated into the pressure sensor 102 and/or the UV-C lighting 103. Furthermore, functionality of a single component may be distributed over multiple components. For example, the controller 101 may comprise a plurality of processors. The controller 101 may be physically separated from the other components of the filter 100. For example, while the other components of the filter need to be physically located in an exhaust duct of a kitchen ventilation system, the controller may be located outside of the kitchen ventilation system. In addition, it should be appreciated that the filter 101 may optionally comprise additional components, such as a housing, filter blades, and/or grease drainage channels.

Although Figure 1 shows that a pressure sensor 102 is used to determine the airflow, alternative sensors for determining airflow are also possible. For example, additionally or alternatively, an optical sensor may be used.

The controller 101 may be implemented using hardware, software, and/or a combination thereof. For example, certain functions of the controller 101 may be carried out by a general purpose computer executing appropriate software.

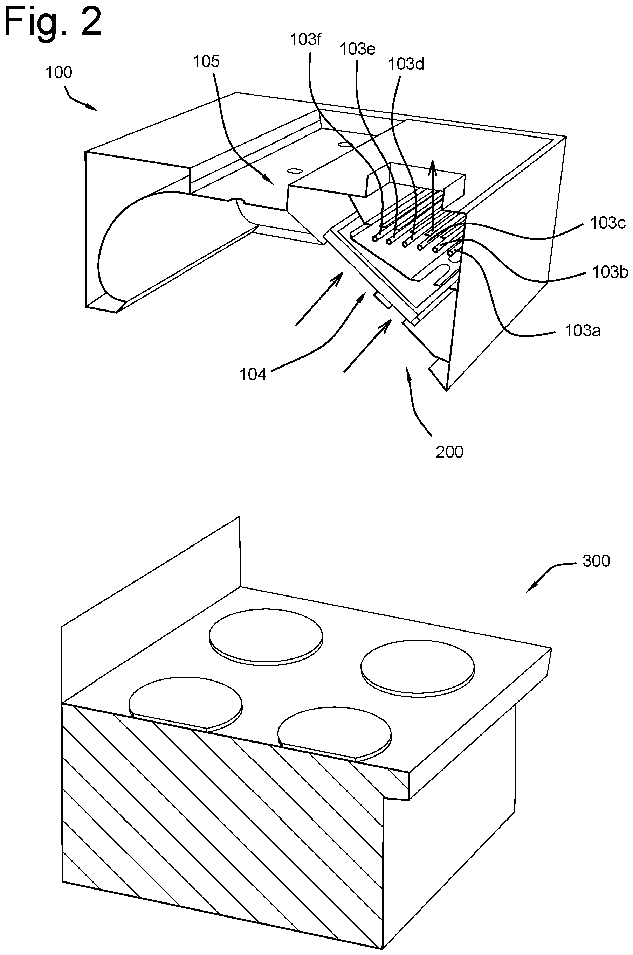

Figure 2 schematically shows an opened-up drawing of a filter 100 in a kitchen ventilation system 200.

The skilled person appreciates that Figure 2 is schematic in nature. Furthermore, Figure 2 is opened-up. In practice, the filter 100 is enclosed in the kitchen ventilation system 200, and the filter 100 in turn has a housing shielding the UV-C lighting 103 from the outside.

Figure 2 shows a filter 100, a kitchen ventilation system 200, and kitchen equipment 300.

Figure 2 shows that the filter 100 comprises UV-C lamps 103a ~ 103f, an air inlet 104 and an air outlet 105. The filter 100 may be similar to the filter 100 shown in Figure 1, and further comprise a controller and an airflow sensor, such as a pressure sensor. The number of UV-C lamps may be varied, as described above. The filter 100 also comprises a housing, and the skilled person appreciates that the housing is generally closed, except for the air inlets 104 and the air outlets 105.

The kitchen ventilation system 200 may be a conventional (professional) kitchen ventilation system. The kitchen equipment may be conventional (professional) kitchen equipment.

The filter 100 may be retro-fitted into an existing kitchen ventilation system 200. In particular, the filter 100 can function essentially independently, and as long as there is sufficient space inside an exhaust of the kitchen ventilation system 200, the filter 100 may be retro-fitted.

For retro-fitting the filter 100, the kitchen ventilation system 200 merely needs to provide an exhaust with sufficient size for the filter 100, as well as a sufficient airflow, even if the installation of the filter causes a slight reduction therein. For example, retro-fitting a filter 100 in an existing kitchen ventilation system exhaust may cause a pressure drop of less than around 200 Pa between the filter air inlet 104 and air outlet 105. It may be advantageous to provide a connection between a controller of the filter 100 and a motor of the kitchen ventilation system 200. In this way, the controller may receive a signal indicating whether the motor (and hence the kitchen ventilation system 200) is operating, as also described above. However, the controller can also determine whether the kitchen ventilation system 200 is operating using an airflow sensor comprised within the filter 100. Because the filter 100 puts few requirements on the kitchen ventilation system 200, it may be retro-fitted into may different kinds of existing kitchen ventilation systems 200, in order to improve filtering therein.

In operation, cooking fumes originate in proximity of the cooking equipment 300. Depending on what is being cooked, these fumes may comprise grease and/or various odours. The kitchen ventilation system 200 is configured to suck up these cooking fumes into an exhaust duct of the kitchen ventilation system 200. The filter 100 is installed in an inside of said exhaust duct. The cooking fumes enter the filter 100 via the air inlet 104. The cooking fumes then remain inside a cavity of the filter 100 for a certain amount of time. The cavity of the filter 100 is dimensioned such that, in operation and on average, the cooking fumes spend at least about 2 seconds or more inside said cavity. The cavity of the filter 100 comprises ozone gas, generated by the UV-C lamps 103a-103f. While the cooking fumes are inside the cavity, the ozone gas chemically reacts with odours in the cooking fumes. Furthermore, the UV-C light causes chemical reactions in the grease, thereby removing at least some the grease from the exhaust air. The reaction products may be drained away. So, after the chemical reaction, the cooking fumes contain no, or at least significantly less, grease and/or odour. The filtered cooking fumes may then leave the filter via the air outlet 105, into a downstream portion of the exhaust duct of the kitchen ventilation system 200.

The amount of ozone produced per time unit depends on the intensity of the UV-C light produced by the UV-C lamps 103a-103f. So, if the intensity of the UV-C light is higher, more ozone is produced.

A maximum intensity of the UV-C light is typically chosen based on a worst-case scenario, in which the kitchen ventilation system 200 is operating with a maximum airflow. In this case, there may be a high amount of grease and/or odour in the kitchen fumes, while the airflow is high, which means that the kitchen fumes spend a relatively short amount of time inside the cavity of the filter. In order to ensure sufficient grease filtering and/or production of ozone, the maximum light intensity should be sufficiently high.

However, the kitchen ventilation system 200 is not always operating at maximum airflow, for example when there is a low amount of grease and/or odour in the kitchen fumes. If the airflow is below the maximum airflow, the kitchen fumes spend a longer time inside the cavity of the filter compared to the case with maximum airflow. This means that there is more time for grease and/or odours to react. In some cases, the concentration of grease and/or odours may also be lower, even if there is more reaction time. This means that not all UV-C light in the cavity may be necessary to sufficiently filter the kitchen fumes.

In this case, operating the UV-C lighting at maximum intensity may lead to unnecessary energy consumption and/or reduced lifetime of the UV-C lighting. Furthermore, if the ozone production is higher than needed, the exhaust air may be contaminated with excess ozone, leading to a reduced quality of the exhaust air. Therefore, it is desirable to limit the ozone production based on the airflow.

As described above, the ozone production may be limited based on the airflow by measuring the airflow and controlling the UV-C light intensity based thereon. For brevity, this description will not be repeated here.

As used herein, the term filter may refer to a device that is configured to filter air and/or other gases. UV-C light may designate light having a wavelength of less than or equal to 280 nm. The term intensity of the UV-C light may refer to an amount of light produced by the UV-C lighting.

An airflow sensor may designate any device suitable for detecting a volume of an airflow generated by the kitchen ventilation system. Such an airflow sensor may, for example, be implemented using a pressure sensor. A pressure sensor may designate a pressure sensor that is configured to detect a pressure generated by the kitchen ventilation system. The term pressure may refer to a pressure generated by the kitchen ventilation system.

The term controller may mean any device, system or part thereof that controls at least one operation. The controller may be implemented in the form of general purpose computing equipment and/or of dedicated hardware. The controller may be configured to execute various steps using a software implementation. The controller may interact with the airflow sensor, the UV-C lighting, and/or with other components of the filter, and/or the kitchen ventilation system, and/or components thereof, for example through signals, such as electrical signals. This interaction may be direct, and/or via additional components.

The term operably connected may mean that two components are configured to exchange digital and/or analogue signals, for example wirelessly or wiredly, either directly or indirectly, for example via a third component.

An adjustable UV-C lighting may designate a lighting system that is configured to produce UV-C light, wherein the intensity of the UV-C light is adjustable. Such an adjustable UV-C lighting system may comprise one or more UV-C lamps, at least some of which may be dimmable and/or individually and/or jointly controllable. A UV-C lamp may designate a lamp that emits UV-C light.

The UV-C lighting may comprise one or more UV-C lamps. The UV-C lamps may be part of the UV-C lighting. Apart from the UV-C lamps, the UV-C lighting may comprise further components, such as, but not limited to, a separate housing, a dedicated power supply, and/or a dedicated controller.

The term first intensity may refer to the first level of light produced by the UV-C lighting. The term second intensity may refer to an intensity of the UV-C light that is higher than or equal to the first intensity. The term maximum intensity may refer to the maximum output of light produced by the UV-C lighting.

The term expected lifetime may refer to the expected life expectancy of one or more UV-C lamps.

The filter may be part of the kitchen ventilation system. The filter may be provided as a part of the kitchen ventilation system upon installation of the kitchen ventilation system and/or retro-fitted into an existing kitchen ventilation system. The kitchen ventilation system may comprise the "filter". Nevertheless, the filter may be provided as a stand-alone unit, to be integrated into an (existing) kitchen ventilation system.

**In** the foregoing description of the figures, the invention has been described with reference to specific embodiments thereof. It will, however, be evident that various modifications and changes may be made thereto without departing from the scope of the invention as summarized in the attached claims.

In addition, many modifications may be made to adapt a particular situation or material to the teachings of the invention without departing from the essential scope thereof. Therefore, it is intended that the invention not be limited to the particular embodiments disclosed, but that the invention will include all embodiments falling within the scope of the appended claims.

In particular, combinations of specific features of various aspects of the invention may be made. An aspect of the invention may be further advantageously enhanced by adding a feature that was described in relation to another aspect of the invention.

It is to be understood that the invention is limited by the appended claims only. In this document and in its claims, the verb "to comprise" and its conjugations are used in their non-limiting sense to mean that items following the word are included, without excluding items not specifically mentioned. In addition, reference to an element by the indefinite article "a" or "an" does not exclude the possibility that more than one of the element is present, unless the context clearly requires that there be one and only one of the elements. The indefinite article "a" or "an" thus usually means "at least one". Terms such as "first" or "second" may simply be used to distinguish the component from other components in question, and do not limit the components in other aspects (such as importance or order).

## Claims

1. Filter (100) for a kitchen ventilation system (200), comprising:
UV-C lighting (103), wherein the UV-C lighting (103) is configured to produce UV-C light, wherein an intensity of the UV-C light is adjustable;
an airflow sensor (102), configured to measure data related to an airflow generated by the kitchen ventilation system (200); and
a controller (100), configured to:
receive a signal from the airflow sensor (102), wherein the signal relates to measurement data regarding an airflow generated by the kitchen ventilation system (200);
based on the signal, determine whether the kitchen ventilation system (200) is operating, and
if the controller (101) determines that kitchen ventilation system (200) is operating:
determine an airflow generated by the operating kitchen ventilation system (200); and
control the UV-C lighting (103) to produce UV-C light, wherein the intensity of the UV-C light is based on the airflow generated by the kitchen ventilation system (200).

2. The filter (100) according to claim 1, wherein the controller (101) is configured to control the UV-C lighting (103) to produce light at a first intensity if the airflow generated by the kitchen ventilation system (200) is below a first predetermined airflow.

3. The filter (100) according to claim 2, wherein the controller (101) is configured to control the adjustable UV-C lighting (103) to produce light at at least a second intensity if the airflow generated by the kitchen ventilation system (200) is above a second predetermined airflow, wherein the second predetermined airflow is greater than or equal to the first predetermined airflow.

4. The filter (100) according to claim 3, wherein the second intensity is less than a maximum intensity of the UV-C lighting (103), wherein the second predetermined airflow is equal to the first predetermined airflow, wherein the controller (101) is further configured to control the adjustable UV-C lighting (103) to produce light at the maximum intensity, when the kitchen ventilation system (200) is generating more than a third predetermined airflow, wherein the third predetermined airflow is greater than the second predetermined airflow.

5. The filter (100) according to any of the preceding claims, wherein the intensity of the UV-C light is proportional to the airflow generated by the kitchen ventilation system (200).

6. The filter (100) according to any of the preceding claims, wherein the UV-C lighting (103) comprises a plurality of UV-C lamps (103a ~ 103d), wherein, when the UV-C lighting (103) produces light at the first intensity, a first fraction of the plurality of UV-C lamps (103a ~103d) is switched on and a first remainder of the plurality of UV-C lamps (103a ~103d) is switched off, and wherein, when the UV-C lighting (103) produces light at the second intensity, a second fraction of the plurality of UV-C lamps (103a ~103d) is switched on and a second remainder of the plurality of UV-C lamps (103a ~103d) is switched off.

7. The filter (100) according to claim 5, wherein the controller (101) is further configured to determine which of the UV-C lamps (103a ~103d) to switch on or off, based on an expected lifetime of the UV-C lamps (103a ~103d).

8. The filter (100) according to any of the preceding claims, wherein the controller (101) is further configured to, if the controller (101) determines that the kitchen ventilation system (200) is not operating, switch off the UV-C lighting (103).

9. The filter (100) according to claim 8, wherein the controller (101) is operably connected to the kitchen ventilation system (200), and wherein the controller (101) is further configured to determine whether the kitchen ventilation system (200) is operating based on a signal received from the kitchen ventilation system (200).

10. Kitchen ventilation system (200), comprising a filter (100) according to any of the preceding claims.

11. Method of filtering kitchen exhaust air, comprising:
receiving a signal from an airflow sensor, wherein the signal relates to measurement data regarding an airflow generated by the kitchen ventilation system;
based on the signal, determining whether a kitchen ventilation system is operating, and
if it is determined that kitchen ventilation system is operating:
determining an airflow generated by the operating kitchen ventilation system; and
controlling UV-C lighting to produce UV-C light, wherein the intensity of the UV-C light is based on the airflow generated by the kitchen ventilation system.

12. The method of claim 11, wherein the method is performed by a filter (100) according to any of claims 1-9.

13. A computer program product, which, when executed on a controller, causes the controller to execute the method according to claim 11 or 12.

14. A memory, having stored thereon the computer program product according to claim 13.
